# EUROPEAN PATENT APPLICATION

(11) **EP 4 029 457 A1**
(43) Date of publication of application: **20.07.2022**
(21) Application number: 21305054.5
(22) Date of filing: 18.01.2021
(51) Int. Cl.: A61B 17/00, A61M 5/315, A61F 2/00

(54) **APPLICATOR, KIT AND METHOD FOR APPLYING AN ADHESIVE COMPOSITION ON A SUPPORT, SUCH AS A TISSUE REPAIR SUPPORT**

(71) Applicant: TISSIUM, 75012 Paris (FR)
(72) Inventor: LOPES, Miguel, 92130 Issy les Moulineaux (FR); PEREIRA, Maria, 1200-366 Lisboa (PT); MOREAU, David, 75010 Paris (FR); BITTON, Elisa, 75012 Paris (FR)
(74) Representative: Regimbeau

(57) **Abstract**

An applicator (1) comprising: a body (2) defining a chamber (14) for storing an adhesive composition, and an outlet (16) for delivering the adhesive composition stored in the chamber (14), a piston (4) mobile in translation relative to the body (2), a drive element (6) mobile in rotation relative to the body (2), and configured to be releasably engaged with a guide (100, 200, 300) external to the applicator (1), wherein rotating the drive element (6) relative to the body (2) causes the piston (4) to translate relative to the body (2) so as to reduce a volume of the chamber (14) and force the adhesive composition out of the applicator (1) through the outlet (16).

## Description

### TECHNICAL FIELD

The present disclosure deals with an applicator, a kit and a method for applying an adhesive composition on a support, such as any substrate having a surface, more particularly a tissue repair support, such as surgical patch, for instance a hernia mesh.

### BACKGROUND

A hernia occurs when an organ, intestine or fatty tissue squeezes through a hole or a weak spot in the surrounding muscle or connective tissue. Hernias often occur at the abdominal wall. Sometimes a hernia can be visible as an external bulge particularly when straining or bearing down.

A hernia mesh is a surgical patch forming a support for supporting damaged tissue around a hernia as it heals. Handling the hernia mesh, poor visibility during mesh handling, implantation and fixation, combined with poor usability and ergonomic limitations when using laparoscopic instrument are challenging tasks for surgeons. Surgeons place the mesh over the hernia region, and the positioned mesh may then be affixed using suitable connecting means such as either stitches, tacks, staples or tissue adhesive in order to prevent its folding or migration.

Fixation using stitches or staples have been reported as leading to nerve injury and acute/chronic postoperative pain. These complications have thus prompted surgeons to evaluate methods of atraumatic fixation such as the use of human fibrin adhesive (for example Tissucol/Tisseel fibrin glue (Baxter Healthcare, Deerfield, IL, USA).

If the adhesive is not correctly applied on the mesh, the mesh may not be conveniently placed and fixed on targeted site. In particular, the surgeon can apply too much adhesive in some areas, or miss adhesive in other areas, which is not desired.

Alternative solution has been developed consisting in self fixating meshes such as Adhesix^{™} that is a mesh coated with a layer of polyvinyl pyrrolidone (PVP) and PEG.

WO2017/195198 further describes a mesh coated with cross-linkable protein or polypeptide such as gelatin with an enzymatic cross-linker such as transglutaminase and US 9,427,298 describes textile implants having a face at least partially covered with a bio-adhesive composition activatable in a moist or wet medium.

In operation, the mesh, including mesh coated with fixating agent (such as adhesive), is preferably inserted using a trocar having a diameter of about 10 mm to 12 mm with the rolled-up mesh placed therein. This may require that said fixating agent is applied onto the mesh using particular patterns, with specific spacings and designs. It is thus desirable to get tools allowing to produce this type of coated supports.

### SUMMARY

An aim of the present disclosure is to ensure that an adhesive composition is applied in reproducible, standardized, uniform and easy manner on a support such as any substrate having a surface, more particularly a tissue repair support, such as surgical patch, surgical mesh or surgical film.

This aim is achieved by an applicator comprising:
- a body defining a chamber for storing an adhesive composition, and an outlet for delivering the adhesive composition stored in the chamber,
- a piston mobile in translation relative to the body,
- a drive element mobile in rotation relative to the body, and configured to be releasably engaged with a guide external to the applicator, wherein rotating the drive element relative to the body causes the piston to translate relative to the body so as to reduce a volume of the chamber and force the adhesive composition out of the applicator through the outlet.

The applicator may comprise the optional feature set forth below, taken alone or combined together whenever it is technically feasible.

The applicator may be configured such that the adhesive composition is forced out of the applicator through the outlet at a rate proportional to a rotational speed of the drive element relative to the body.

The outlet may be arranged to deliver the adhesive composition in a direction parallel to a rotation axis of the drive element relative to the body.

The piston may be mobile in translation relative to the body in a direction parallel to a rotation axis of the drive element relative to the body.

The body may define a passageway connecting the chamber with the outlet, wherein the drive element extends around the passageway.

The drive element may comprise a pinion able to mesh with a rack external to the applicator.

The applicator may comprise a gripping part allowing a user to hold and the applicator, and wherein the drive element is located between the outlet and the gripping part.

The applicator may further comprise a first screw thread, and the drive element may comprise a second screw thread engaged with the first screw thread, wherein a rotation of the second screw thread relative to the first screw thread causes the piston to translate relative to the body.

The applicator may comprise: a gripping part allowing a user to hold the applicator, and a pusher comprising the first screw thread, the pusher being slidably mounted to the gripping part.

The applicator may comprise blocking means preventing the body to rotate relative to the pusher about an axis of translation of the piston relative to the body.

The body and the piston may form a syringe being a replaceable part of the applicator.

The applicator may further comprise the adhesive composition.

The adhesive composition may be a light-curable compound.

A kit for applying an adhesive composition on a support may comprises a guide and the applicator as described above. The applicator and the guide are able to be set in an engaged configuration wherein the drive element is engaged with the guide and wherein the outlet faces the support, and wherein moving the drive element along the guide causes the drive element to rotate relative to the body while the guide and the applicator are in the engaged configuration.

The guide may comprise a rack able to mesh with the drive element when the guide and the applicator are in the engaged configuration.

The guide may define an elongated hole leaving exposed an elongated area of the support whenever the guide covers the support.

The applicator may comprise a mouthpiece defining the outlet and being able to extend in the elongated hole whenever the applicator and the guide are in the engaged configuration.

The guide may comprise a guiding wall extending along the elongated hole and such that the outlet is aligned with the elongated hole when the drive element is moved along the guiding wall while the guide and the applicator are in the engaged configuration.

The guiding wall may comprise a rack able to mesh with the drive element when the guide and the applicator are in the engaged configuration.

The elongated hole may extend over at least 180 degrees around a point and/or forms a portion of a loop extending around a point.

The guide may define a plurality of holes including the first elongated hole, the plurality of holes forming a loop extending around a point.

The guide may define a further plurality of holes forming a second loop extending around a point, wherein the loop and the second loop are concentric.

The guide may comprise a central hole leaving exposed another area of the support while the guide covers the support, and the point may be the center of the central hole.

The guide may comprise a second guiding wall extending along the elongated hole, and wherein the drive element is between the guiding wall and the second guiding wall whenever the guide and the applicator are in the engaged configuration.

The guide may define a second elongated hole leaving exposed a second elongated area of the support when the guide covers the support, and wherein the guide comprises a second guiding wall extending along the second elongated hole and arranged such that the outlet is aligned with the second elongated hole when moving the drive element along the second guide wall while the applicator the guide and are in the engaged configuration.

The second guiding wall may be between the elongated hole and the second elongated hole.

The kit may further comprise the support, and the support may be a surgical mesh, such as a hernia mesh.

The kit may further comprise a container containing the support and the guide assembled together.

It is also proposed a method for applying an adhesive composition on a support using the kit described above. The method comprises: setting the applicator and the guide in the engaged configuration, and moving the drive element along the guide so as to causes the drive element to rotate relative to the body, while the guide and the applicator are in the engaged configuration.

### BRIEF DESCRIPTION OF THE FIGURES

Further details, features and advantages are explained in more detail below with the aid of exemplary embodiments that are illustrated in the figures.
- Figure 1 is a view in perspective of an applicator according to a first embodiment.
- Figure 2 is a side view of the applicator depicted in figure 1.
- Figure 3 is a view in perspective of a body of the applicator depicted in figure 1.
- Figure 4 is a cross-sectional view of a drive element of the applicator depicted in figure 1.
- Figure 5 is a cross-sectional view of a gripping part of the applicator depicted in figure 1.
- Figure 6 is a view in perspective of the gripping part depicted in figure 5.
- Figure 7 shows different parts of the applicator depicted in figure 1.
- Figure 8 is a cross-sectional view on the line A-A shown in figure 2.
- Figure 9 is a top view of a kit including the applicator depicted in figure 1 and a guide according to a first embodiment.
- Figure 10 is a view in perspective of the kit depicted in figure 9.
- Figure 11 is a cross-sectional view of the kit depicted in figure 9.
- Figure 12 is a top view of a kit including the applicator depicted in figure 1 and a guide according to a second embodiment.
- Figure 13 is a view in perspective of the kit depicted in figure 12.
- Figure 14 is a top view of a kit including an applicator equivalent to the one depicted in figure 1 and a guide according to a third embodiment.
- Figure 15 is a bottom view of an applicator according to a second embodiment.
- Figure 16 is a perspective view of a kit comprising an applicator according to a third embodiment and the guide according to the third embodiment.
- Figure 17 is another view of the kit of figure 16, showing the interior of the applicator according to the third embodiment.
- Figure 18 is a perspective view of a kit comprising an applicator according to a fourth embodiment and the guide according to the third embodiment.
- Figure 19 is another view of the kit of figure 16, showing the interior of the applicator according to the fourth embodiment.

### DETAILED DESCRIPTION OF EMBODIMENTS

### 1) Applicator

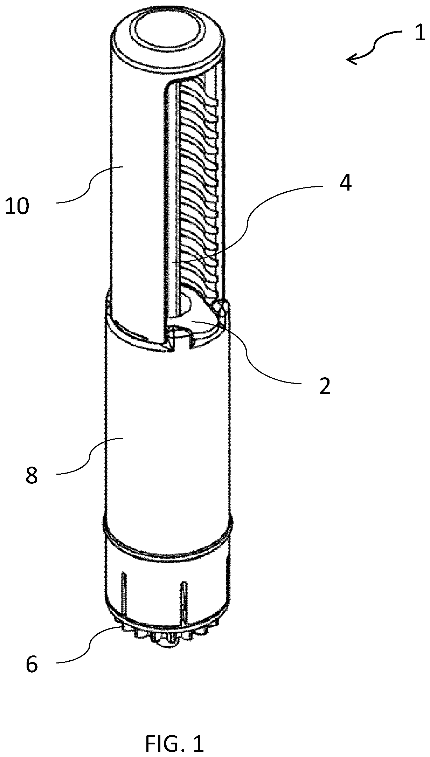

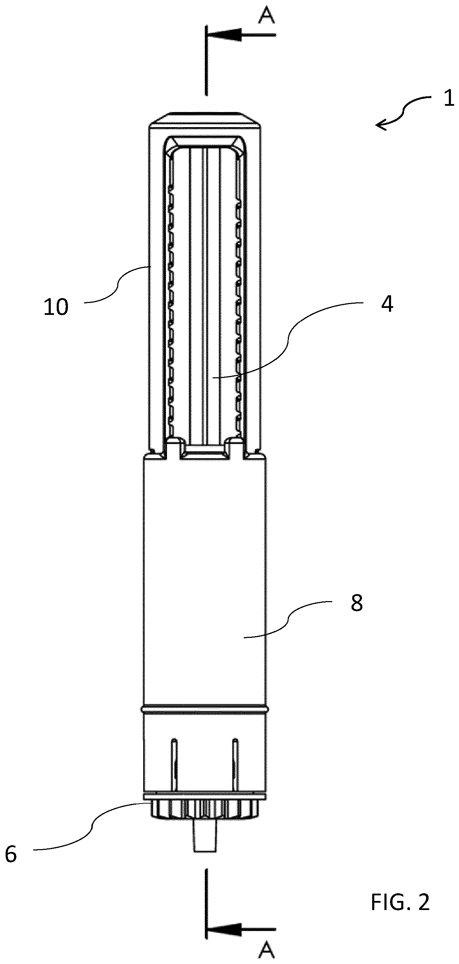

Referring to **figures 1** **and** **2****,** an applicator 1 according to an embodiment comprises a body 2, a piston 4, a drive element 6, a gripping part 8 and a pusher 10.

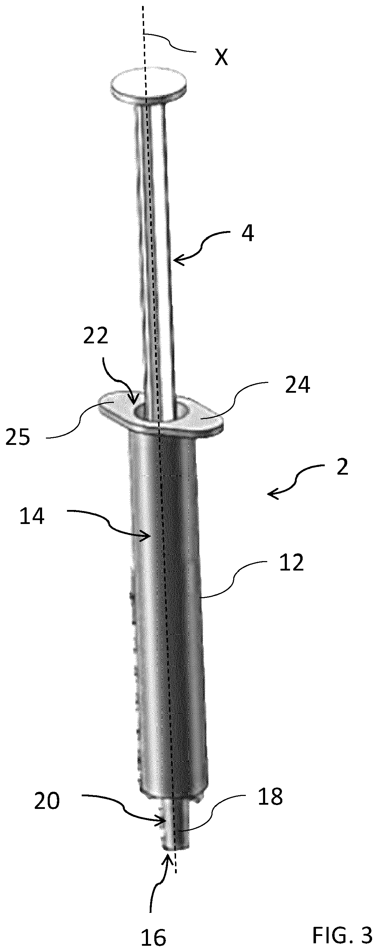

As shown with more details in **figure 3****,** the body 2 is an elongated body extending along a longitudinal axis X from a distal end thereof to a proximal end thereof.

The body 2 comprises a cylinder 12 defining a chamber 14 for storing an adhesive composition (this composition will be detailed later). The chamber 14 may have a volume varying from 0,1 to 500 ml, preferably from 1 to 50 ml, for instance 3 ml.

The body 2 further comprises an outlet 16 for delivering the adhesive composition stored in the chamber 14 out of the applicator 1.

The outlet 16 is arranged axially at the distal end of the body 2. Thus, the adhesive composition flows in a direction parallel to longitudinal axis X when exiting the applicator via the outlet 16.

The body 2 further comprises a mouthpiece 18 defining a passageway 20 connecting the chamber 14 with the outlet 16. The outlet 16 actually forms a distal opening of the passageway 20 (and of the applicator 1).

The mouthpiece 18 has a smaller diameter than that of the cylinder 12.

The chamber 14, the outlet 16 and the passageway 20 extend along the longitudinal axis X. The passageway 20 is rectilinear. The passageway is located between the chamber 14 and the outlet 16. Thus, an adhesive composition stored in chamber 14 has to flow in a direction parallel to longitudinal axis X so as to reach the outlet 16 and exit the applicator 1.

The body 2 further has an inlet 22 opposite to the outlet 16 relative to the chamber 14. The inlet 22 is defined by the cylinder 12 at the proximal end of the body 2.

The body 2 further comprises two tabs 24, 25 protruding radially from an outer surface of the cylinder 12. The tabs 24, 25 are opposite to each other relative to the cylinder 12.

The piston 4 is mobile in translation relative to the body 2 in a direction coaxial to longitudinal axis X. The piston 4 actually delimits the chamber 14 when inserted in the cylinder 12 through the inlet 22. When the piston 4 translates relative to the body 2 towards the outlet 16, the volume of the chamber 14 is reduced; this causes an adhesive composition stored in the chamber 14 to exit the chamber 14, then flow in the passageway 20 then be forced out of the applicator 1 through the outlet 16. When the piston 4 is moved away from the outlet 16, the volume of the chamber 14 is increased.

The body 2 and the piston 4 form a medical device such as a syringe. This syringe is a replaceable part of the applicator 1 independent from the drive element 6, the gripping part 8 and the pusher 10. Thus, it can be replaced by another syringe.

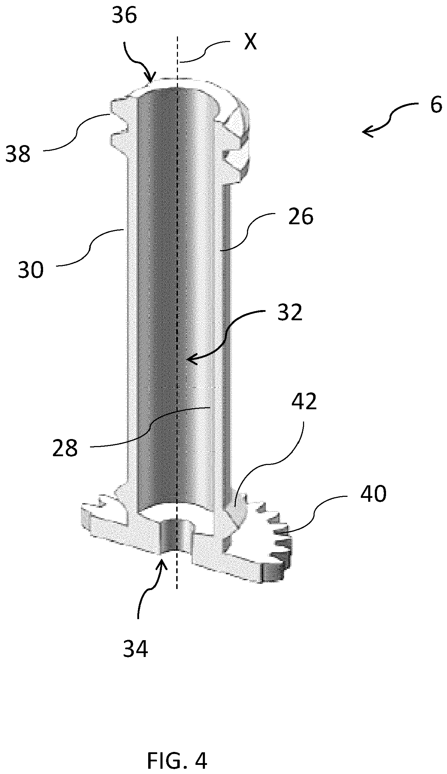

Now referring to **figure 4****,** the drive element 6 comprises a tube 26 extending about longitudinal axis X.

The tube 26 has an inner surface 28 and an outer surface 30 opposite to the inner surface 28. The inner surface 28 delimits a cylindrical cavity 32.

The tube 26 has two opposite openings giving access to the cavity 32: a distal opening 34 and a proximal opening 36. The cylinder 12 can be inserted in the cavity 32 of the tube 26 via the proximal opening 36, and positioned therein such that the mouthpiece 18 crosses the distal opening 34 and protrudes out of the drive element 6.

The drive element 6 further comprises a screw thread 38. The screw thread 38 is formed in the outer surface 30 of the tube 26. The screw thread 38 extends from the proximal opening 36.

The drive element 6 further comprises a wheel 40 affixed to the tube 26. The wheel 40 and the tube 26 are coaxial (about longitudinal axis X). The wheel 40 forms a collar around the distal opening 34 and around the tube 26. The wheel 40 has therefore a diameter greater than the diameter of the outer surface 30.

The distal opening 34 is defined in the wheel 40.

The distal opening 34 has a diameter smaller than that of the cylinder 12. Thus, when the body 2 is inserted in the tube 26, the cylinder 12 abuts against the wheel 40.

In the embodiment shown in figure 4, the wheel 40 is actually a pinion. The pinion 40 comprises teeth on its outer circumference.

The drive element 6 further comprises a rib 42 protruding from the outer surface 30. The rib 42 is located between the screw thread 38 and the pinion 40. The rib may form a collar extending around the tube 26.

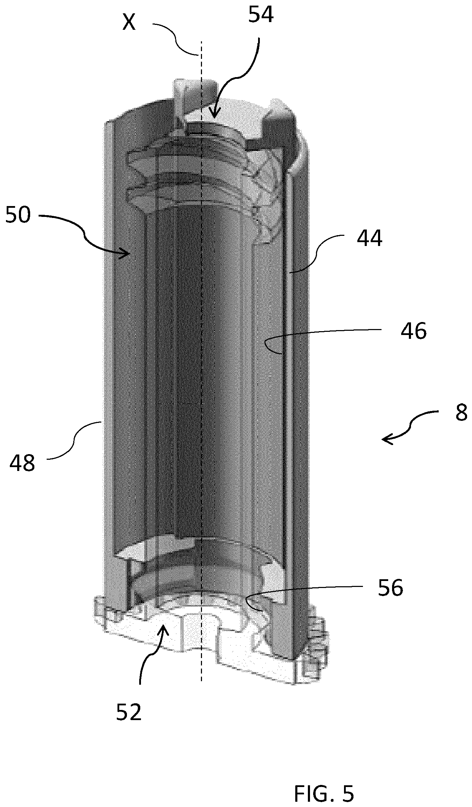

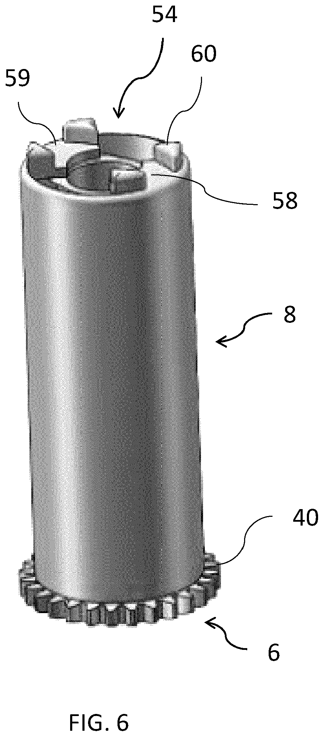

Referring to **figures 5** **and** **6****,** the gripping part 8 of the applicator 1 comprises a tube 44 extending about longitudinal axis X.

The tube 44 has an inner surface 46 and an outer surface 48 opposite to the inner surface 46. The inner surface 46 delimits an inner cavity 50. The outer surface 48 is a free surface of the applicator 1. The outer surface can be seized by a hand of a user.

The tube 44 has having two opposite openings giving access to the cavity 50: a distal opening 52 and a proximal opening 54.

The tube 26 of the drive element 6 can be inserted in the second tube 44 via the distal opening 52, such that both tubes are coaxial and can freely rotate relative to each other about their common axis. As a result, the drive element 6 is rotatably mounted on the gripping part 8.

The inner surface 46 defines a recess 56 able to receive the rib 42 when the tube 26 of the drive element 6 is inserter in the tube 44 of the gripping part 8. The recess 56 may form a groove extending around the cavity 50. The recess 56 and the rib 42 form locking means preventing the drive element 6 to translate relative to the gripping part 8.

The diameter of the inner surface 46 is greater than the diameter of the outer diameter 30 of the tube 26, so as to leave an annular gap in the cavity 50 between the tubes 26, 44. In particular, the screw thread 38 extends this annular gap.

The gripping part 8 further comprises two tabs 58, 59 protruding radially from the inner surface 46. The two tabs 58, 89 actually define therebetween the proximal opening 54.

The proximal opening 54 has a central portion and two peripheral portions opposite to each other relative to the central portion. The central portion is circular and has a diameter substantially equal to the outer diameter of the cylinder 12, and substantially equal to the diameter of inner surface 28.

The gripping part 8 further comprises a plurality of pawns 60 protruding from the tabs 58, 59 in a direction parallel to the axis of the tube 44. The plurality of pawns 60 include two first pawns protruding from tab 58 and a two second pawns protruding from tab 59. The two first pawns and tab 58 form a first recess able to receive tab 24, and the two second pawns and tab 59 forms a second recess able to receive tab 25 (or vice-versa).

The drive element 6 is locked in the gripping part 8 by means of the recess 56 and the rib 42. The central portion of the proximal opening 54 is aligned with the proximal opening 36 of the drive element 6. Besides, the wheel 40 remains out of the gripping part 8. Thus, the wheel can engage a guide external to the applicator 1 (this guide will be detailed later). Besides, the drive element 6 can freely rotate relative to the gripping part 8.

The body 2 can then be inserted into the gripping part 8 via its proximal opening, then into the drive element 6 locked therein. During this insertion, the body 2 reaches a position wherein the tabs 24, 25 of the body 2 abut against the tabs 58, 59 of the gripping parts 8, in the two recesses defined by the plurality of paws 60. As a result, tabs 24, 25, 58, 59 and pawns 60 form blocking means preventing the body 2 to rotate relative to the gripping part 8. But alternate blocking means playing this function could be used.

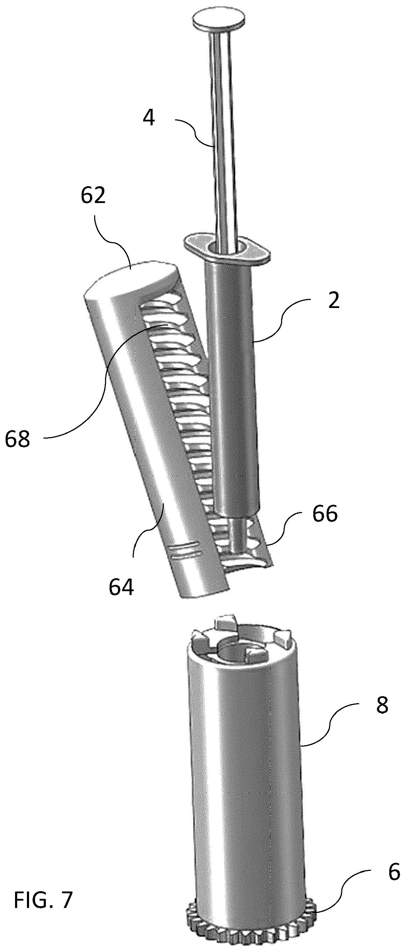

Referring to **figure 7****,** the pusher 10 comprises a base 62 and two legs 64, 66 protruding from the base 62. The legs 64, 66 comprise two inner surfaces facing each other, are and separated by a space. The piston is received in this space.

The pusher 10 comprises a screw thread 68. The screw thread 68 is formed in the inner surfaces of the two legs 64, 66.

Once the body 2 has been locked to the gripping part 8 and the piston 4 has been inserted in the cylinder 12, the two legs 64, 66 are inserted via the two peripheral portions of the proximal opening 54 in the annular gap left between the gripping part 8 and the drive element 6, such that the screw thread 68 engages with the screw thread 38. The tabs 58, 59 form means preventing the pusher to rotate relative to the gripping part 8 (other means for carrying out this function could be used instead). As a result, the pusher 10 is slidably mounted in the gripping part 8. A rotation of the screw thread 38 relative to the screw thread 68 cause the pusher 10 to translate relative to the gripping part 8 in a direction parallel to longitudinal axis X.

As shown in figure 1, the gripping part 8 and the pusher 10 form together a housing containing the piston 4 and the body 2. The pusher 10 prevents the piston 4 to be moved out of the body 2, since the base 62 abuts against the piston 4.

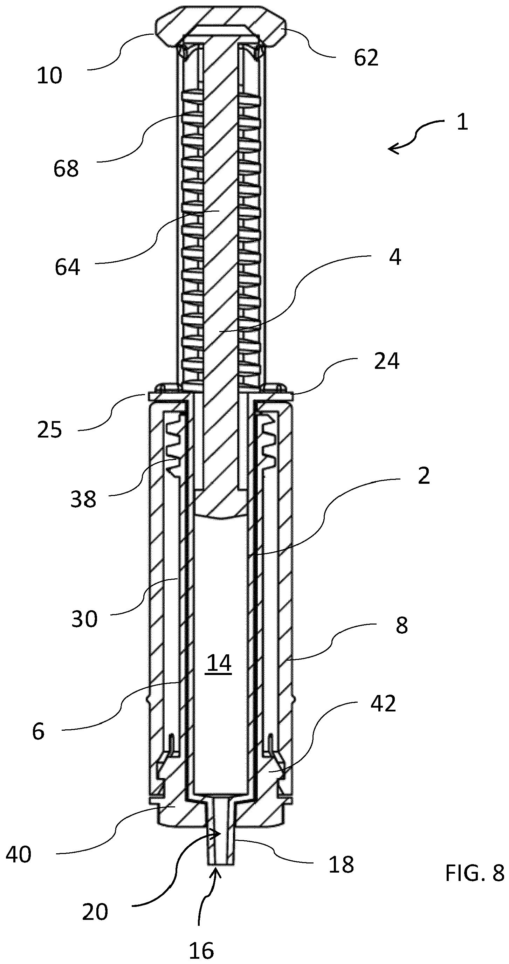

Once, the body 2, the piston 4, the drive element 6, the gripping part 8 and the pusher 10 have been assembled as described above, the applicator 1 is formed, as shown in figure 8.

### 2) Adhesive composition

The chamber 14 is filled with an adhesive composition.

Preferably, the adhesive composition is a light-curable compound. "Light curable compound" refers to compounds that are configured to polymerize or otherwise cure upon receiving radiant energy, more particularly in the form of light from a light source.

The light-curable compound may comprise a pre-polymer and a photoinitiator, said photoinitiator being able to induce polymerization of the said pre-polymer when exposed to light of a specific wavelength.

According to a special embodiment, said photoinitiator is sensitive to ultraviolet (UV) radiations.

Examples of suitable pre-polymers include, but are not limited to pre-polymers described in US10,179,195 or WO2016/202984 or WO2016/202985.

Preferably, the polymeric backbone of the pre-polymer comprises a polymeric unit of the general formula (-A-B-)n, wherein A is derived from a substituted or unsubstituted polyol or mixture thereof and B is derived from a substituted or unsubstituted polyacid or mixture thereof; and n represents an integer greater than 1. The polymeric backbone is made up of repeating monomer units of general formula -A-B-. The term "substituted" has its usual meaning in chemical nomenclature and is used to describe a chemical compound in which a hydrogen on the primary carbon chain has been replaced with a substituent such as alkyl, aryl, carboxylic acid, ester, amide, amine, urethane, ether, or carbonyl. Component A of the pre-polymer may be derived from a polyol or mixture thereof, such as a diol, triol, tetraol or greater. Suitable polyols include diols, such as alkane diols, preferably octanediol; triols, such as glycerol, trimethylolpropane, trimethylolpropane ethoxylate, triethanolamine; tetraols, such as erythritol, pentaerythritol; and higher polyols, such as sorbitol. Component A may 5 also be derived from unsaturated polyols, such as tetradeca-2,12-diene-1,14-diol, polybutadienediol or other polyols including macromonomer polyols such as, for example polyethylene oxide, polycaprolactone triol and N-methyldiethanoamine (MDEA) can also be used. Preferably, the polyol is substituted or unsubstituted glycerol. Component B of the pre-polymer is derived from a polyacid or mixture thereof, preferably diacid or triacid. Exemplary acids include, but are not limited to, glutaric acid (5 carbons), adipic acid (6 carbons), pimelic acid (7 carbons), sebacic acid (8 carbons), azelaic acid (nine carbons) and citric acid. Exemplary long chain diacids include diacids having more than 10, more than 15, more than 20, and more than 25 carbon atoms. Non-aliphatic diacids can also be used. For example, versions of the above diacids having one or more double bonds can be used to produce polyol-diacid copolymers. Preferably the polyacid is substituted or unsubstituted sebacic acid.

Examples of suitable photoinitiators sensitive to UV radiations include, but are not limited to: 2-dimethoxy-2-phenyl-acetophenone, 2-hydroxy-1-[4-(hydroxyethoxy)phenyl]-2-methyl-1-propanone (Irgacure 2959), 1-hydroxycyclohexyl-1-phenyl ketone (Irgacure 184), 2-hydroxy-2-methyl-1-phenyl-1-propanone (Darocur 1173), 2-benzyl-2-(dimehylamino)-1-[4-morpholinyl) phenyl]-1-butanone (Irgacure 369), methylbenzoylformate (Darocur MBF), oxyphenyl-acetic acid-2-[2-oxo-2-phenyl-acetoxy-ethoxy]-ethyl ester (Irgacure 754), 2-methyl-1-[4-(methylthio)phenyl]-2-(4-morpholinyl)-1-propanone (Irgacure 907), diphenyl(2,4,6-trimethylbenzoyl)-phosphine oxide (Darocur TPO), phosphine oxide, phenyl bis(2,4,6-trimethyl benzoyl) (Irgacure 819), and combinations thereof.

According to another embodiment, said photoinitiator is sensitive to visible light (typically blue light or green light).

Examples of photoinitiators sensitive to visible light include, but are not limited to: diphenyl(2,4,6-trimethylbenzoyl)-phosphine oxide, eosin Y disodium salt, N-Vinyl-2-Pyrrolidone (NVP) and triethanolamine, and camphorquinone.

Alternatively, the "adhesive composition" might be any composition which have flow characteristics such that they can be applied to the desired area through a syringe or catheter (e.g., relatively low viscosity) but are sufficiently viscous to remain in place at the site of application.

### 3) Guide

### 3.1) First embodiment

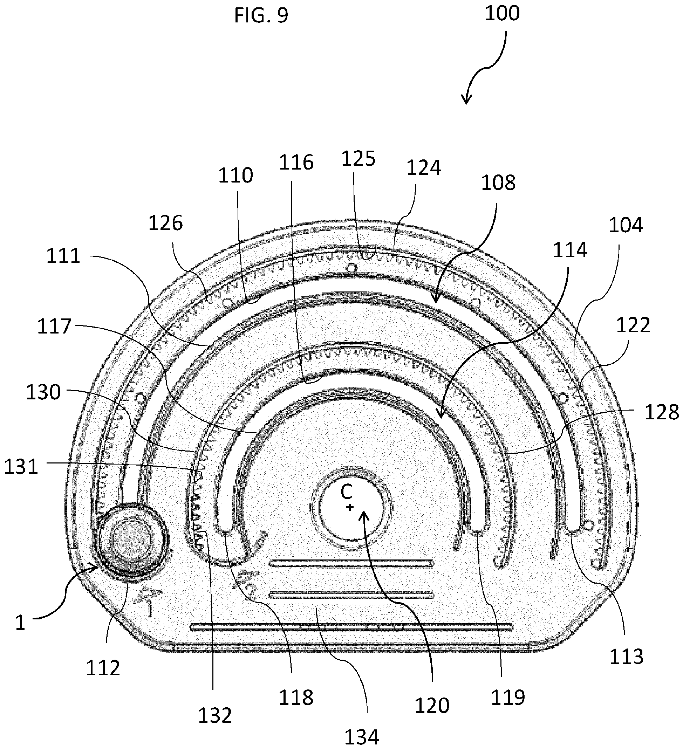

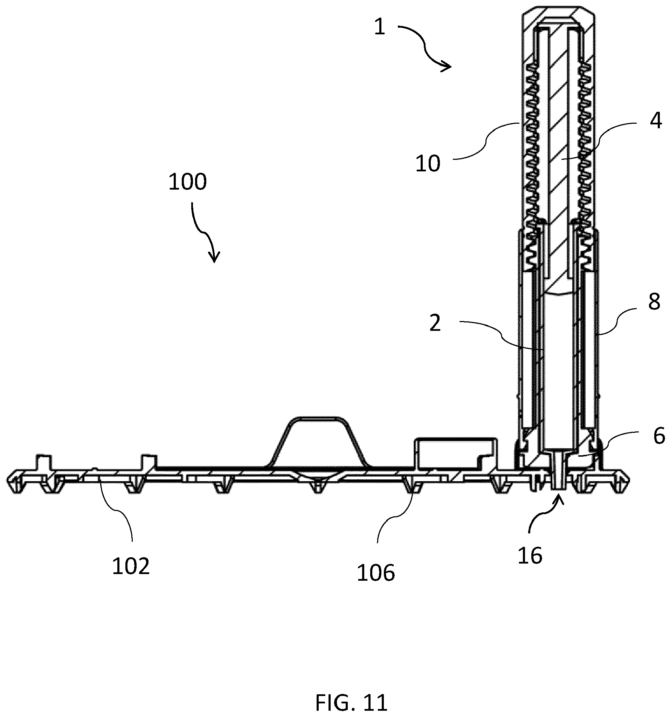

Referring to **figures 9****,** **10** **and** **11****,** a kit for applying an adhesive composition on a support such as any substrate having a surface, more particularly a tissue repair support, such as surgical patch, preferably hernia mesh, comprises the applicator 1 and a guide 100 according to a first embodiment.

The guide 100 comprises a lower surface 102 and an upper surface 104 opposite to the lower surface 102. The lower surface 102 is intended to be put in contact with the support.

The guide 100 comprises stabilizing means for preventing the guide 100 to slide against the support. These stabilizing means may for instance comprise protrusions 106 protruding from the lower surface 102. When the support is a mesh, such as a surgical mesh, these protrusions can engage or pick the loosely woven body of mesh thereby preventing this sliding movement.

The guide 100 defines a first elongated hole 108 opening in both the lower surface 102 and the upper surface 104. As a consequence, once the lower surface 102 of the guide 100 is placed against a support, the first elongated hole 108 leaves exposed a first elongated area of the support.

The first elongated hole 108 has a first lateral edge 110, a second lateral edge 111 opposite to the first lateral edge 110. The first elongated hole 108 has a constant width measured as a distance separating the two lateral edges 110, 111.

The first elongated hole 108 has two opposite ends 112, 113 connecting the two lateral edges 110, 111. The two opposite ends 112, 113 are curved such that the first elongated hole 108 is oblong.

The first elongated hole 108 forms a portion of a first ring having a center C. In other words, the two lateral edges 110, 111 are arcuate. The first lateral edge 110 has a radius of curvature greater than that of the second lateral edge 111.

The first elongated hole 108 extends over at least 180 degrees about the center C. In the first embodiment, the first elongated hole 108 extends over an angular sector close to 180 degrees about center C, typically less than 190 degrees.

The guide 100 further defines a second elongated hole 114 opening in both the lower surface 102 and the upper surface 104. As a consequence, once the lower surface 102 of the guide 100 is placed against one of the two surfaces of the hernia mesh, the second elongated hole 114 leaves exposed another elongated area of the support.

The second elongated hole 114 has a first lateral edge 116 and a second lateral edge 117 opposite to the first lateral edge 116. The second elongated hole 114 has a constant width measured as a distance separating the two lateral edges 116, 117.

The second elongated hole 114 has two opposite ends 118, 119 connecting the two lateral edges 116, 117. The two opposite ends 118, 119 are curved such that the second elongated hole 114 is oblong.

The second elongated hole 114 forms a portion of a second ring. In other words, the two lateral edges 116, 117 are arcuate. The first lateral edge 116 has a radius of curvature greater than that of the second lateral edge 117.

The second elongated hole 114 extends over at least 180 degrees about the center C. In the first embodiment, the second elongated hole 114 extends about center C over an angular sector close to 180 degrees, typically less than 190 degrees.

The two portions of ring formed by the first elongated hole 108 and the second elongated hole 114, respectively, are concentric (they have the same center C).

The guide 100 further comprises a central hole 120. The second elongated hole 114 is between the central hole 120 and the first elongated hole 108. The first elongated hole 108 surrounds the second elongated hole 114, and the second elongated hole 114 surrounds the central hole 120.

The center of the central hole 120 is actually the center C of the two rings above mentioned.

The guide 100 further comprises a first guiding wall 122 extending along the first elongated hole 108. The first guiding wall 122 protrudes from the upper surface 104.

The first lateral edge 110 is closer to the first guiding wall 122 than the second lateral edge 111. The first guiding wall 122 is away from the first elongated hole 108 in the sense that there is a non-zero distance between the first lateral edge 110 of the first elongated hole 108 and the first guiding wall 122. This distance is constant all along the first lateral edge 110.

The first guiding wall 122 has a first lateral surface 124, a second lateral surface 125 opposite to the first lateral surface 124, and a top surface joining the two lateral surfaces together. The second lateral surface 125 faces a first zone located above the first elongated hole 108.

The first guiding wall 122 comprises a first rack 126 able to mesh with the pinion 40. The first rack 126 is formed in the second lateral surface 125 of the first guiding wall 122, such that the rotation axis X of the pinion can be perpendicular to the lower surface 102 of the guide 100 when the pinion 40 meshes with the first rack 126.

The guide 100 further comprises a second guiding wall 128 extending as well along the second elongated hole 114. The second guiding wall 128 protrudes from the upper surface 104.

The first elongated hole 108 is between the first guiding wall 122 and the second guiding wall 128.

The first guiding wall 122 and the second guiding wall 128 define therebetween a first groove. The first zone discussed above is located in this first groove.

The pinion 40 can fit in the first groove. For that purpose, the first groove has a width, measured as a distance between the first guiding wall 122 and the second guiding wall 128, which is greater or equal to the diameter of the pinion. When the pinion is meshes with the first rack 126, the outlet 16 faces the first elongated hole 108 and the pinion is away from the second guiding wall 128.

The first elongated hole 108 is closer to the first guiding wall 122 than to second guiding wall 128.

The second lateral edge 111 of the first elongated hole 108 may be closer to the second guiding wall 128 than the first guiding wall 122. The first guiding wall 122 is away from the second elongated wall in the sense that there is non-zero distance between the second lateral edge 111 of the first elongated hole 108 and the second guiding wall 128. This distance is roughly constant all along the second lateral edge 111.

The second guiding wall 128 has a first lateral surface 130, a second lateral surface 131 opposite to the first lateral surface, and a top surface joining the two lateral surfaces together. The first lateral surface 130 faces the first zone above the first elongated hole 108, and faces as well the second lateral surface 125 of the first guiding wall 122.

Besides, the second guiding wall 128 is between the first elongated hole 108 and the second elongated hole 114.

The first lateral edge 116 of the second elongated hole 114 is closer to the second guiding wall 128 than the second lateral edge 117. The second guiding wall 128 is away from the second elongated hole 114 in the sense that there is non-zero distance between the first lateral edge of the second elongated hole 114 and the second guiding wall 128. This distance is roughly constant all along the first lateral edge.

The second guiding wall 128 comprises a second rack 132, which is able to mesh with the pinion 40. The second rack 132 is formed in the second lateral surface 131 of the second guiding wall 128. Thus, the teeth of the rack face a second zone located above the second elongated hole 114.

Besides, the second the second elongated hole 114 is between the second guiding wall 128 and the central hole 120.

The guide 100 further comprises a gripping part 134 away from the guiding walls 122, 128 and away from the holes 108, 114, 120. A user can maintain the guide 100 against a support by pressing on the gripping part 134 without covering the guiding wall 122, 128 or the holes 108, 114, 120.

The guide 100 may be obtained by injection molding or 3D printing.

The guide has a height measure in a direction perpendicular to the lower and upper surface, which is preferable less than 5 centimeters.

### 3.2) Second embodiment

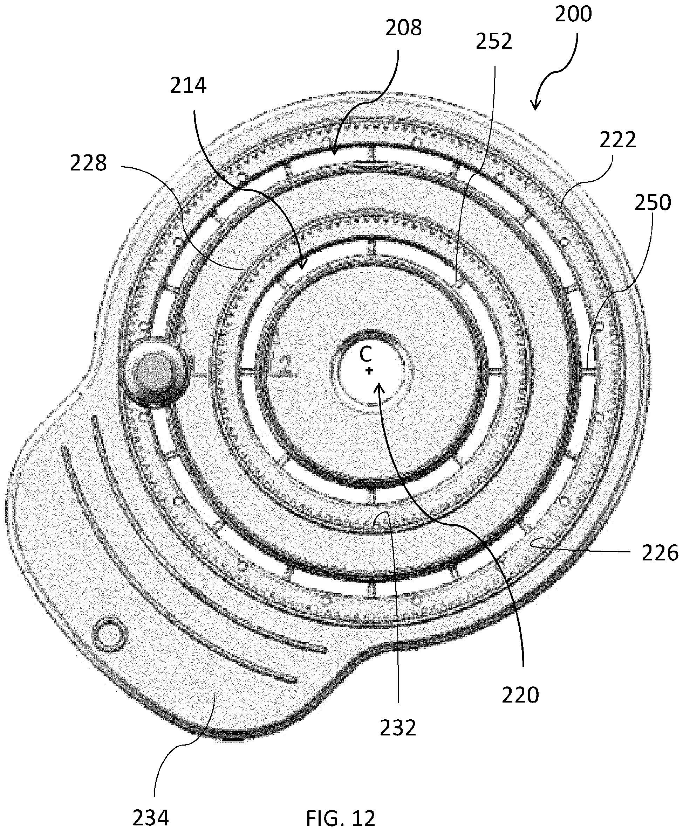

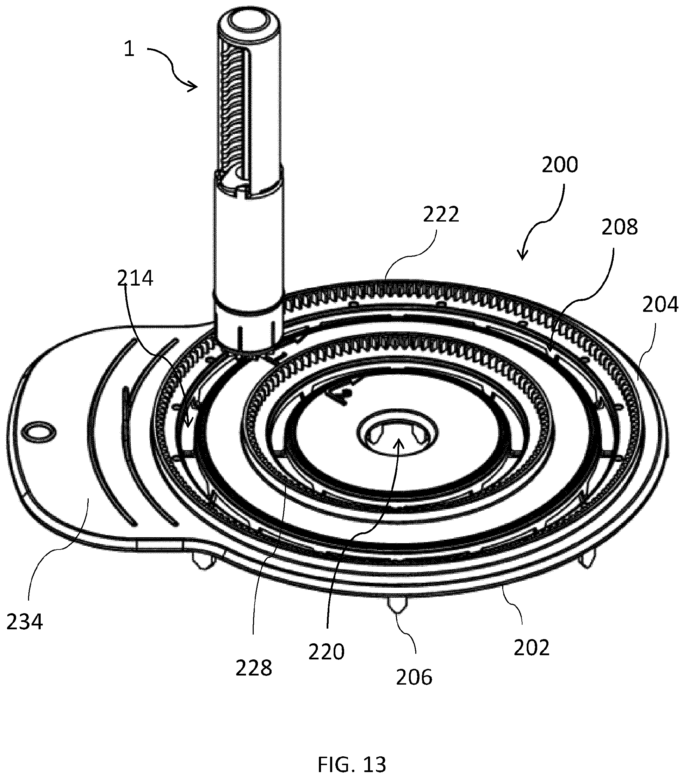

A guide 200 according to a second embodiment is illustrated in **figures 12** **and** **13****.**

The guide 200 comprises features corresponding to features of the guide 100 described above. By convention, the reference numerals of two corresponding features in guides 100, 200 differ by 100. Thus, the guide 200 comprises the following features:
- A lower surface 202 and an upper surface 204.
- Stabilizing means including protrusions 206.
- A plurality of first elongated holes 208, a plurality of second elongated holes 214, and a central hole 220 having a common center C.
- A first guiding wall 222 including a first rack 226, and a second guiding wall 228 including a second rack 232, both racks 226 and 121 being able to mesh with the pinion 40.
- A gripping part 234.

Guide 200 differs from guide 100 by the number and shape of the elongated holes, and by the shape of the guiding walls. All other features of guide 200 are similar to the corresponding features of guide 100.

The first elongated holes 208 are distributed around the center C, so as to form together a first loop. The loop is interrupted by first bridges 250. Each first bridge 250 extends radially with respect to center C, and separates two adjacent first elongated holes 208. Due to the presence of the first bridges, the plurality of first elongated holes 208 extends over an angular sector about center C which is not strictly equal to 360 degrees. Nevertheless, the first bridges 250 have a small width, such that this angular sector is close to 306 degrees, typically greater than 320 degrees. In the embodiment shown in **figure 12****,** the number of first bridges 250 is sixteen, but it could be different (two or more).

Similarly, the second elongated holes 214 are distributed around the center C, so as to form together a second loop included in the first loop. The second loop is interrupted by second bridges 252. Each second bridge 252 extends radially with respect to center C, and separates two adjacent second elongated holes 214. Due to the presence of the second bridges 252, the plurality of second elongated holes 214 extends over an angular sector about center C which is not strictly equal to 360 degrees. Nevertheless, the second bridges 252 have a small width, such that this angular sector is close to 360 degrees, typically greater than 320 degrees. In the embodiment shown in **figure 12****,** the number of second bridges 252 is eight, but it could be different (two or more).

Besides, the first guiding wall 222 and the second guiding wall 228 form two other loops extending over 360 degrees about center C. Racks 226 and 232 form two closed circuits extending as well over 360 degrees about center C.

In the guide 200 shown in **figure 12****,** the loops formed by the elongated holes 208, 214 and by the guiding walls 222, 228 form two rings (circular loops). But in other embodiments, these loops could have other closed shapes such as ellipsoids or polygons extending around the central hole 220.

### 3.3) Third embodiment

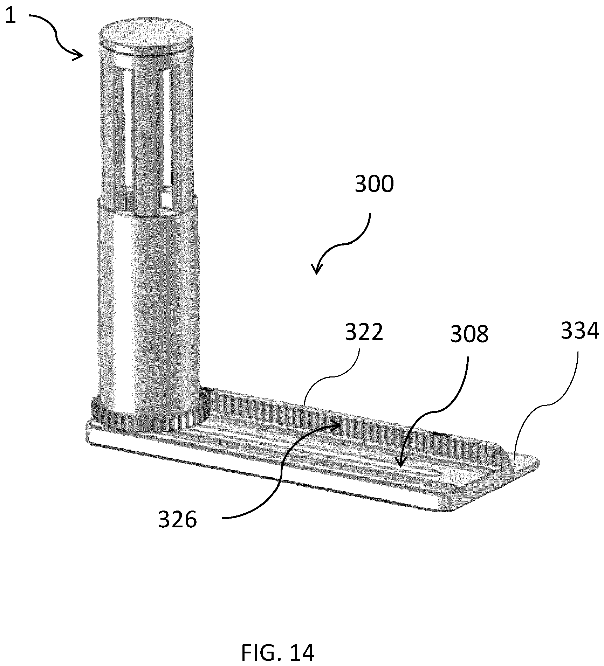

A guide 300 according to a third embodiment is illustrated in **figures 14****.**

Guide 300 differs from guide 100 in that it has a single elongated hole 308 and a single guiding wall 322 extending along hole 308. The guiding wall includes a rack 326.

Moreover, the elongated hole 308, the guiding wall 322 and the rack 326 are rectilinear.

Guide 300 further comprises a gripping part 334 extending along the guiding wall 322.

### 4) Further components of the kit

The present disclosure provides a kit comprising an applicator 1 and any of guides 100, 200 and 300.

According to preferred embodiment, the kit comprises a body 2 and the chamber 14 thereof is filled with an adhesive composition.

The kit including the applicator 1 and any of guides 100, 200, 300 may further include a support on which the adhesive composition can be applied.

The support has a top surface able to be partially covered by any of the guides 100, 200, 300. It may have any shape (circular, rectangular, etc.).

The support is for example any substrate having a surface, more particularly a tissue repair support, such as surgical patch, a surgical mesh, such as a hernia mesh. The mesh is a screen-like material that is used as a reinforcement for tissue, organ or bone. It can be made of synthetic polymers or biopolymers. Materials used for surgical mesh include:
- Non-absorbable synthetic polymers (polypropylene).
- Absorbable synthetic polymers (polyglycolic acid or polycaprolactone).
- Biologic (acellular collagen sourced from cows or pigs).
- Composite (a combination of any of the three previous materials).
- The mesh is known per se.

The kit including the applicator 1 and any of guides 100, 200, 300 may further include container, such as a blister. This container can contain the guide 100 or 200 or 300 preassembled with a support, such as any substrate having a surface, more particularly a tissue repair support, such as surgical patch, a surgical mesh a surgical mesh. This container serves different purposes:
- Sterilizing the guide 100 or 200 or 300 on top of the support,
- Having the guide and the support perfectly aligned with each other,
- Hydrate the support.

### 5) Method for applying the adhesive composition on the support

### 5.1) Method using the applicator 1 and the guide 100

A first method for applying the adhesive composition on a support using the applicator 1 and the guide 100 comprises the following steps. In the following, it will be assumed that the support is a hernia mesh, which is a particular type of surgical mesh.

A user covers the mesh with the guide, such that the lower surface of the guide contacts an upper surface of the mesh. For this point on, the first elongated hole 108 leaves exposed a first elongated area of the mesh, the second elongated hole 114 leaves exposed a second elongated area of the mesh, and the central hole 120 leaves exposed a circular area of the mesh, whereas other areas of the mesh are masked by the guide.

The chamber 14 of the applicator is filled with the adhesive composition discussed above. This step can be a preliminary step performed before assembling the body 2 with the other parts of the applicator 2.

The user then seizes the gripping part 8 of the applicator 1 filled with the adhesive composition.

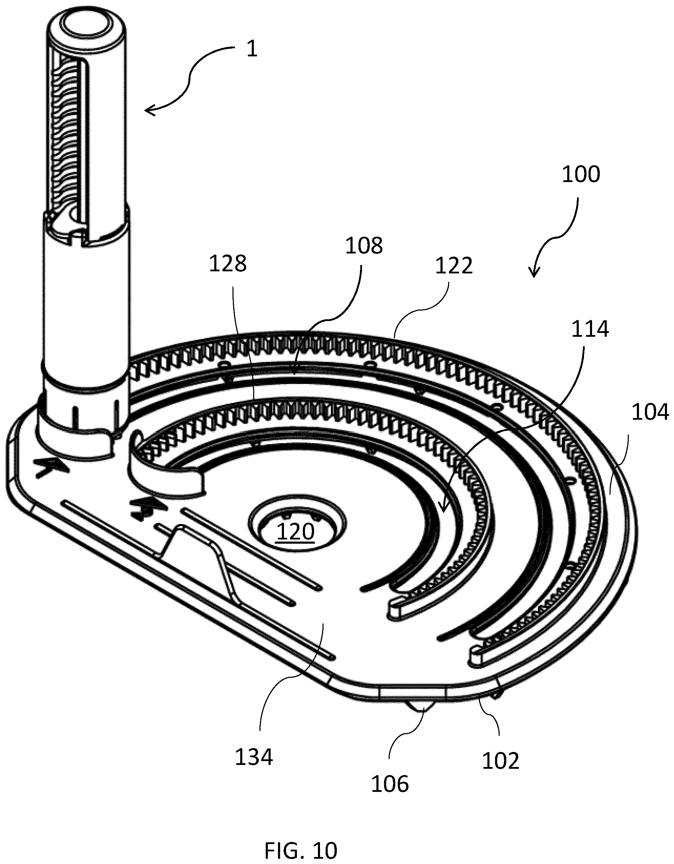

The user sets the guide and the applicator in a first engaged configuration, which is shown for example in **figure 10****.**

In the first engaged configuration, the drive element 6 is engaged with the guide 100. More precisely, the pinion 40 is in the first groove between the first guiding wall 122 and the second guiding wall 128, and engages the first rack 126.

In the first engaged configuration, the outlet 16 faces a first elongated area of the mesh left exposed by the first elongated hole 108. The mouthpiece 18 extends in the first elongated hole 108.

While the applicator 1 and the guide 100 are in the first engaged configuration, the user translates the applicator 1 relative to the guide 100 along the first guiding wall 122. During this translation, the outlet 16 travels the first elongated area and the pinion 40 rotate relative to the rack 126. The coupling between the first rack 126 and the pinion 40 causes the drive element 6 to rotate relative to the gripping part 8, the pusher 6 and the body 2. The rotation of the drive element 6 relative to the pusher 6 causes the pusher 6 to translate relative to the body 2 in a direction parallel to longitudinal axis X. During this translation, the pusher 6 pushes on the piston 4, whereby the volume of the chamber 14 is reduced. The adhesive composition stored in the chamber 14 flows in the passageway 20, then is forced out of the applicator 1 through the outlet 16. Since the outlet 16 simultaneously travels the first elongated area, the adhesive composition is gradually and evenly applied on the first elongated area.

Thanks to the design of the applicator 1, the adhesive composition is forced out of the applicator through the outlet 16 at a flow rate proportional to the rotational speed of the drive element relative to the body. This property is very advantageous since the adhesive composition can be applied evenly on the mesh even though the user does not translate the applicator 1 relative to the guide 100 at a constant speed. Thus, the adhesive composition is evenly deposited on the mesh with ease.

The ratio between the rate and the rotational speed depend on various parameters including the diameter of the pinion 40 and the width of the chamber 14. Preferably, the flow rate is comprised between 0,04 g/cm and 0,08 g/cm.

The user moves the applicator 1 such that the outlet 16 moves from one end of the first elongated hole 108 to an opposite end of the first elongated hole 108. As a result, the entirety of the first elongated area of the mesh is coated with the adhesive composition.

During this process, the guide 100 acts as a stencil. The adhesive composition is only applied in the first elongated area, having a predefined pattern, which can be reproduced very easily.

During the step described above, the user can use the central hole 120 as a visor. The user can for instance draw a mark on the mesh at the center C of the central hole 120 using a tool such as a pen. Any movement of the mark in the hole warns the user that the guide 100 moves relative to the mesh.

Then the user repeats the steps described above so as to coat the second elongated area of the mesh with the adhesive composition. For this purpose, the user sets the guide 100 and the applicator in a second engaged configuration, wherein the pinion 40 is engages the second rack, and wherein the outlet faces the second elongated hole 114 and the second elongated area of the mesh left exposed by the second elongated hole 114. While the applicator 1 and the guide 100 are in the second engaged configuration, the user translates the applicator 1 relative to the guide 100 along the second guiding wall 128. The user moves the applicator 1 such that the outlet moves from one end of the second elongated hole 114 to an opposite end of the second elongated hole 114. As a result, the entirety of the second elongated area of the mesh is coated with the adhesive composition.

Then, the user moves the guide 100 away from the mesh, thereby revealing the adhesive composition applied selectively in the first elongated area and the second elongated area.

The first elongated area and the second elongated area extend in a first half of the mesh.

The user folds a second half of the mesh back on the first half around a fold line crossing the mark the user has previously drawn at the center C while the guide 100 covered the mesh. By doing so, the adhesive composition is transferred to the second half of the mesh so as to form two concentric loops of adhesive on the support once the mesh is unfolded.

However, the mesh is not immediately unfolded to reveal the two concentric loops.

The folded mesh is rolled in a "taco" like shape then grabbed with graspers), and is then inserted in the body of a patient (e.g. through laparoscopic trocar), for instance inside the abdominal cavity. The mesh is then unrolled and unfolded and placed on the abdominal wall so as to cover a hernia thereon.

When the adhesive composition is a light-curable compound, the adhesive composition is illuminated with light such that the adhesive composition polymerizes. As a result, the adhesive composition can adhere on the abdominal wall.

### 5.2) Second method using the applicator 1 and the guide 200

A second method for applying the adhesive composition on the support uses the applicator 1 and the guide 200 instead of guide 100. The second method comprises the same steps as the first method. The only difference lies in the fact that the user can move the applicator 1 at 360 degrees about the center C along guiding walls 222, 228, so as to form two loops of adhesive composition on the support, instead of two portions of loops.

An advantage of the second method over the first method is that folding the support back on itself is no more necessary to form loops of adhesives on the support, it is no longer required to have uniform and symmetric coating on the mesh surface.

### 5.3) Third method using the applicator 1 and the guide 300

A third method for applying the adhesive composition on the support uses the applicator 1 and the guide 300 instead of guides 100 or 200. The third method comprises the same steps as the first method. The only difference lies in the fact that the user can move the applicator along guiding wall 322, so as to form a line of adhesive composition on the support.

### 6) Other embodiments

The present disclosure is not limited by the embodiments described above in relation with the figures.

Although these features are advantageous, the wheel 40 of the drive element is not necessarily a pinion, and the guiding walls do not necessarily include racks. The wheel could have a smooth circumference adhering and rolling on a smooth surface.

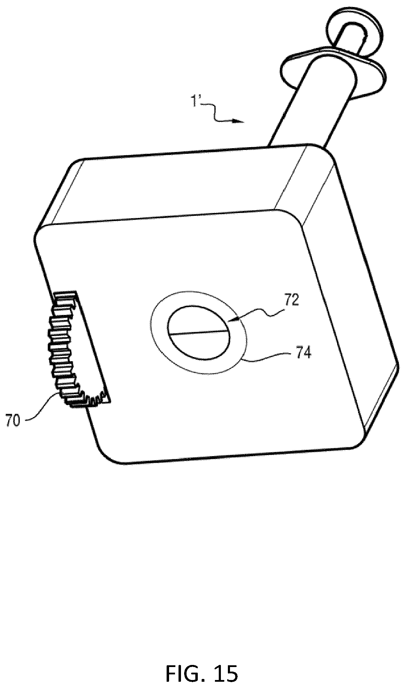

The axis of rotation of the wheel 40 could be different. It could for instance be perpendicular to longitudinal axis X, such that the wheel 40 can roll on the upper surface of any of guides 100, 200 or 300, or directly on the surface of the support on which the adhesive composition is to be applied. In the latter case, the support can be used directly as a guide, which means that using an external guide such as guide 100 or guide 200 is no more necessary. Figure 15 illustrates an applicator 1' which could replace applicator 1 in any of the methods described above. Applicator 1' comprise a wheel 70 able to roll on the surface of the support, so as to cause an adhesive composition stored therein to be forced out of the applicator 1' through an outlet 74. A valve 72 made of a flexible material such as rubber may be arranged at outlet 74 so as to close it. The valve is urged to open outlet 74 under the effect of the adhesive composition, when the applicator 1' rolls on a surface.

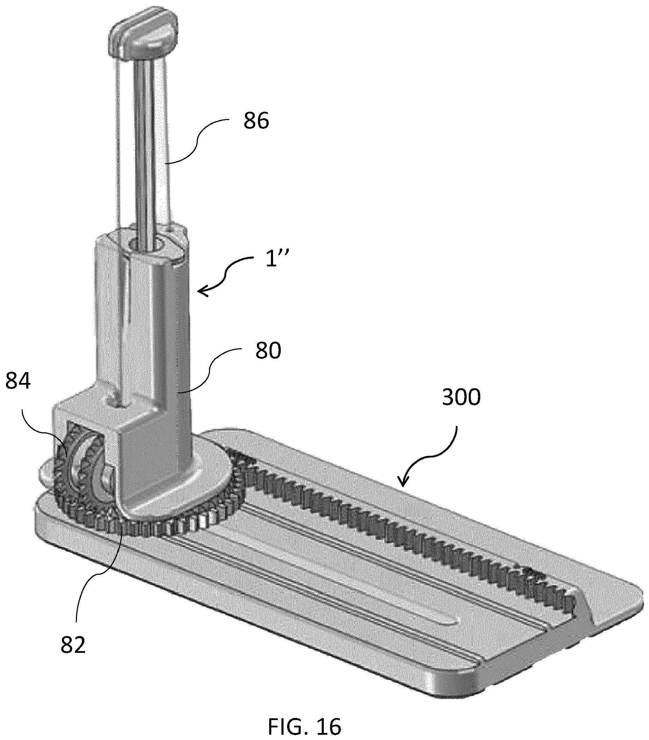

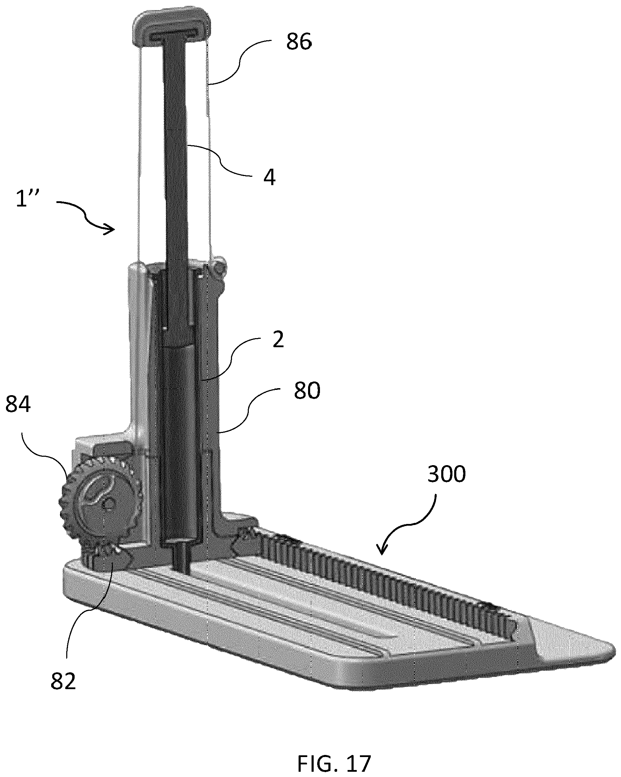

In the applicator 1, the transmission causing the rotational motion of the wheel 40 to be converted into a translation motion of the piston 4 actually includes screw threads 38, 68, which makes the applicator 1 very efficient and robust. Nevertheless, other transmissions, for example including a pulley, a thread and/or a belt could be used instead. For example, **figures 16** **and** **17** illustrate an alternate applicator 1" which could replace applicator 1 in any of the method described above. Applicator 1" comprises: the body 2, the pusher 4, a gripping part 80 playing the same function as gripping part 8, a wheel 82 playing the same function as wheel 40, a gear 84 and a thread 86. The gear 84 comprises a drum. The thread 86 has a first end attached to gear 84 and a second end opposite to the first end and attached to the gripping part 80. A rotation of the wheel 82 relative to the gripping part 80 causes the thread 86 to wrap around the drum and urge the pusher 4 to slide in the body 2.

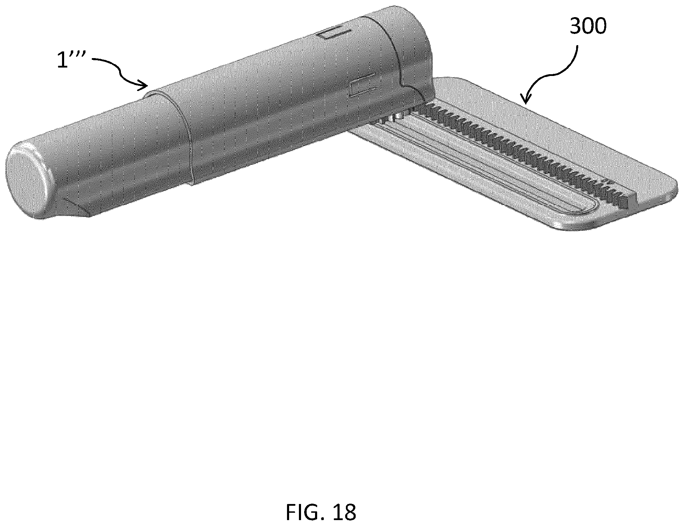

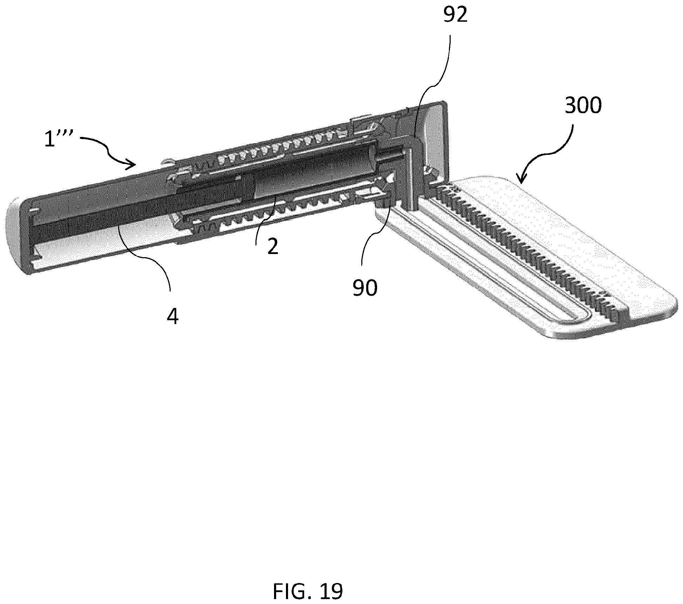

The applicator 1 is supposed to be perpendicular to the support while applying the adhesive composition on the support. This is due to the fact that the chamber, the passageway and the outlet are aligned along longitudinal axis X. This feature is advantageous, but not mandatory. The passageway could for instance be bent at 90 degrees, and such that the chamber extends horizontally when the applicator is set in the engaged configuration. **Figures 18** **and** **19** illustrates an alternate applicator 1"' implementing this and which could replace applicator 1 in any of the method described above. More specifically, applicator 1"' comprise the body 2 and the pusher 4 discussed above. Applicator 1"' further comprise a wheel 90 playing the same function as wheel 40 of applicator 1. Wheel 90 has an axis of rotation perpendicular to the axis of translation of the pusher 4 relative to the body 2. Furthermore, applicator further comprises a passageway 92 which is bent at 90 degrees and arrange to extend the passageway 20 defined by body 2.

The guides 100, 200 could have a different number of elongated holes. Guide 100 could have a single arcuate elongated hole. Guide 200 could be designed to apply a single loop of adhesive, rather than two. The central hole is advantageous since it can be used as a visor, but it is not mandatory.

Of course, any of the applicators described above can be used to apply any type of adhesive composition on any support (patches, films, biologic tissues, and so on).

## Claims

1. An applicator (1) comprising:
• a body (2) defining a chamber (14) for storing an adhesive composition such as a light-curable compound, and an outlet (16) for delivering the adhesive composition stored in the chamber (14),
• a piston (4) mobile in translation relative to the body (2),
• a drive element (6) mobile in rotation relative to the body (2), and configured to be releasably engaged with a guide (100, 200, 300) external to the applicator (1), wherein rotating the drive element (6) relative to the body (2) causes the piston (4) to translate relative to the body (2) so as to reduce a volume of the chamber (14) and force the adhesive composition out of the applicator (1) through the outlet (16).

2. The applicator (1) of claim 1, configured such that the adhesive composition is forced out of the applicator (1) through the outlet (16) at a rate proportional to a rotational speed of the drive element (6) relative to the body (2).

3. The applicator (1) of any one of claims 1 to 2, wherein the piston (4) is mobile in translation relative to the body (2) in a direction parallel to a rotation axis of the drive element (6) relative to the body (2).

4. The applicator (1) of any of claims 1 to 3, wherein the drive element (6) comprises a pinion (40) able to mesh with a rack external to the applicator (1).

5. The applicator (1) of any of claims 1 to 4, comprising a gripping part (8) allowing a user to hold and the applicator (1), and wherein the drive element (6) is located between the outlet (16) and the gripping part (8).

6. The applicator (1) of any of claims 1 to 5, further comprising a first screw thread (68), and wherein the drive element (6) comprises a second screw thread (38) engaged with the first screw thread, and wherein a rotation of the second screw thread relative to the first screw thread (68) causes the piston (4) to translate relative to the body (2).

7. The applicator (1) of claim 6, further comprising:
• a gripping part (8) allowing a user to hold the applicator (1),
• a pusher (10) comprising the first screw thread (68), the pusher being slidably mounted to the gripping part (8).

8. The applicator (1) of claim 7, comprising blocking means preventing the body (2) to rotate relative to the pusher about an axis of translation of the piston (4) relative to the body (2).

9. The applicator (1) of any one of claims 1 to 8, wherein the body (2) and the piston (4) form a syringe, the syringe being a replaceable part of the applicator (1).

10. Kit for applying an adhesive composition on a support such as a surgical mesh, wherein the kit comprises a guide (100, 200, 300) and an applicator (1) according to any one of claims 1 to 9, wherein:
• the applicator (1) and the guide (100, 200, 300) are able to be set in an engaged configuration wherein the drive element (6) is engaged with the guide and wherein the outlet (16) faces the support,
• moving the drive element (6) along the guide (100, 200, 300) causes the drive element (6) to rotate relative to the body (2) while the guide and the applicator (1) are in the engaged configuration.

11. Kit of claim 10, wherein the guide (100, 200, 300) comprises a rack (125, 225, 325) able to mesh with the drive element (6) when the guide and the applicator (1) are in the engaged configuration.

12. Kit of any one of claims 10 and 11, wherein the guide (100, 200, 300) defines an elongated hole (108, 208, 308) leaving exposed an elongated area of the support whenever the guide covers the support.

13. Kit of claim 12, wherein the guide (100, 200, 300) comprises a guiding wall (122, 222, 322) extending along the elongated hole (108, 208, 308) and such that the outlet (16) is aligned with the elongated hole (108, 208, 308) when the drive element (6) is moved along the guiding wall (122, 222, 322) while the guide and the applicator (1) are in the engaged configuration.

14. Kit of claim 13, wherein the guiding wall (122, 222, 322) comprises a rack (125, 225, 325) able to mesh with the drive element (6) when the guide and the applicator (1) are in the engaged configuration.

15. Kit of any one of claims 12 to 14, wherein the guide (200) defines a plurality of holes including the first elongated hole, the plurality of holes forming a loop extending around a point (C).
